(19) Europäisches Patentamt
European Patent Office
Office européen des brevets

(11) EP 2 748 311 B1

(12) EUROPEAN PATENT SPECIFICATION

(45) Date of publication and mention of the grant of the patent:
**16.09.2015 Bulletin 2015/38**

(21) Application number: **12753399.0**

(22) Date of filing: **24.08.2012**

(51) Int Cl.:
*C12N 9/04* (2006.01) *C12Q 1/00* (2006.01)
*C12Q 1/26* (2006.01) *C12Q 1/54* (2006.01)

(86) International application number:
**PCT/EP2012/003572**

(87) International publication number:
**WO 2013/026575 (28.02.2013 Gazette 2013/09)**

# (54) GLUCOSE OXIDASE

GLUKOSEOXIDASE

GLUCOSE OXYDASE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **25.08.2011 EP 11006939**
**27.03.2012 EP 12002193**

(43) Date of publication of application:
**02.07.2014 Bulletin 2014/27**

(73) Proprietors:
- **Roche Diagnostics GmbH**
**68305 Mannheim (DE)**
Designated Contracting States:
**DE**
- **F.Hoffmann-La Roche AG**
**4070 Basel (CH)**
Designated Contracting States:
**AL AT BE BG CH CY CZ DK EE ES FI FR GB GR HR HU IE IS IT LI LT LU LV MC MK MT NL NO PL PT RO RS SE SI SK SM TR**
- **Ultizyme International Ltd.**
**Meguro**
**Tokyo 152-0013 (JP)**

(72) Inventors:
- **KOJIMA, Katsuhiro**
**Tokyo 1520013 (JP)**
- **MORI, Kazushige**
**Tokyo 1520013 (JP)**
- **SODE, Koji**
**Tokyo 183-8538 (JP)**

(74) Representative: **Jung, Michael et al**
**Roche Diagnostics GmbH**
**Patent Department (LMP.....6164)**
**Sandhofer Strasse 116**
**68305 Mannheim (DE)**

(56) References cited:
**EP-A1- 1 892 529** **US-A1- 2004 053 425**

- **BANKAR SANDIP B ET AL: "Glucose oxidase - An overview", BIOTECHNOLOGY ADVANCES, vol. 27, no. 4, July 2009 (2009-07), pages 489-501, XP002695901, ISSN: 0734-9750, DOI: 10.1016/J.BIOTECHADV.2009.04.003**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 748 311 B1

## Description

### Technical Field

The present invention relates to a glucose oxidase for use in a kit and a sensor for the measurement of glucose. More particularly, the present invention relates to a mutant glucose oxidase having reduced oxidase activity.

### Background Art

The concentration of glucose in blood is quite important in clinical tests for diagnosis of diabetes mellitus and in blood-sugar control of patients suffering from diabetes mellitus. The glucose concentration in blood may be measured using an enzyme having specificity to glucose.

Glucose oxidases have been isolated from various kinds of strains and it has been suggested that glucose may be analyzed using such enzymes.

Glucose oxidase is a FAD-dependent enzyme which catalyzes a reaction where glucose is oxidized to generate gluconolacton, with generating the reduced form of FAD (FADH2). FADH2 in turn transmits electron to an electron acceptor and is converted to its oxidized form. In the presence of oxygen, FADH2 preferentially transmits electrons to the oxygen molecule rather than to artificial electron acceptors (also referred to as mediators or electron mediators). Thus, when glucose is assayed by glucose oxidase with mediators, the assay results will be greatly affected by the dissolved oxygen level in the reaction system. Such a disadvantage will be particularly noted in clinical tests of blood samples by a point-of-care testing device utilizing an artificial electron acceptor. The enzymes used for the enzyme sensor strips that employ artificial electron mediators desirably have low activity toward oxygen.

Accordingly, the object of the present invention is to provide an enzyme, in particular a glucose oxidase, whose activity is less affected by the dissolved oxygen level.

### Disclosure of the Invention

The inventors of the present application have been able to provide an enzyme, in particular a glucose oxidase, whose activity is less affected by the dissolved oxygen level. More specifically, this has been achieved by reducing the oxidase activity of an enzyme that in its wild-type form predominantly shows an oxidase activity and by preferably at the same time increasing the enzyme’s dehydrogenase activity. As will be described in more detail below, this has been achieved by mutating the wild type enzyme.

The present inventors have prepared various mutants of a glucose oxidase and surprisingly found that a certain type of mutants exhibit reduced oxidase activity while substantially retaining dehydrogenase activity, in particular dye-mediated dehydrogenase activity.

The present invention provides a mutant glucose oxidase as defined in independent claim 1. Preferred embodiments of the mutant glucose oxidase are the subject matter of the claims depending from claim 1.

In particular, the present invention concerns a mutant glucose oxidase modified at one or more positions selected from:

(a) a position corresponding to the position 53 of the amino acid sequence set forth in SEQ ID NO: 1 by substituting the amino acid residue Thr with another amino acid residue;
(b) a position corresponding to the position 116 of the amino acid sequence set forth in SEQ ID NO: 1 by substituting the amino acid residue Ile with another amino acid residue;
(c) a position corresponding to the position 132 of the amino acid sequence set forth in SEQ ID NO: 1 by substituting the amino acid residue Ser or Thr with another amino acid residue;
(d) a position corresponding to the position 134 of the amino acid sequence set forth in SEQ ID NO: 1 by substituting the amino acid residue Thr with another amino acid residue;
(e) a position corresponding to the position 237 of the amino acid sequence set forth in SEQ ID NO: 1 by substituting the amino acid residue Ile or Phe with another amino acid residue;
(f) a position corresponding to the position 371 of the amino acid sequence set forth in SEQ ID NO: 1 by substituting the amino acid residue Val or Ala with another amino acid residue;
(g) a position corresponding to the position 373 of the amino acid sequence set forth in SEQ ID NO: 1 by substituting the amino acid residue Phe with another amino acid residue;
(h) a position corresponding to the position 434 of the amino acid sequence set forth in SEQ ID NO: 1 by substituting the amino acid residue Glu with another amino acid residue;
(i) a position corresponding to the position 436 of the amino acid sequence set forth in SEQ ID NO: 1 by substituting the amino acid residue Phe with another amino acid residue;

(j) a position corresponding to the position 448 of the amino acid sequence set forth in SEQ ID NO: 1 by substituting the amino acid residue Trp with another amino acid residue; and
(k) a position corresponding to the position 537 of the amino acid sequence set forth in SEQ ID NO: 1 by substituting the amino acid residue Trp with another amino acid residue.

More particularly, the mutant glucose oxidase of the present invention is modified at one or more positions selected from:

(a) a position corresponding to the position 53 of the amino acid sequence set forth in SEQ ID NO: 1 by substituting the amino acid residue Thr with Ser;
(b) a position corresponding to the position 116 of the amino acid sequence set forth in SEQ ID NO: 1 by substituting the amino acid residue Ile with Val;
(c) a position corresponding to the position 132 of the amino acid sequence set forth in SEQ ID NO: 1 by substituting the amino acid residue Ser with Ala, Thr, Val, Cys or Ile, or by substituting the amino acid residue Thr with Ala, Ser, Val, Trp or Cys;
(d) a position corresponding to the position 134 of the amino acid sequence set forth in SEQ ID NO: 1 by substituting the amino acid residue Thr with Ala, Ile or Met;
(e) a position corresponding to the position 237 of the amino acid sequence set forth in SEQ ID NO: 1 by substituting the amino acid residue Ile with Val, or by substituting the amino acid residue Phe with Ile, Ala, Val, Met, Ser, Asp, Leu, Thr, Asn, Arg or Cys;
(f) a position corresponding to the position 371 of the amino acid sequence set forth in SEQ ID NO: 1 by substituting the amino acid residue Val with Thr, Ala, or by substituting the amino acid residue Ala with Val;
(g) a position corresponding to the position 373 of the amino acid sequence set forth in SEQ ID NO: 1 by substituting the amino acid residue Phe with Leu, Tyr, Ala, Met, Asn or Trp;
(h) a position corresponding to the position 434 of the amino acid sequence set forth in SEQ ID NO: 1 by substituting the amino acid residue Glu with Gln;
(i) a position corresponding to the position 436 of the amino acid sequence set forth in SEQ ID NO: 1 by substituting the amino acid residue Phe with Trp, Ala, Leu, Tyr, Met, Glu or Ile;
(j) a position corresponding to the position 448 of the amino acid sequence set forth in SEQ ID NO: 1 by substituting the amino acid residue Trp with Ala, Ile, Ser, Val, Met, Thr, Cys, Gly, Leu, Asn, Asp, Lys, Phe, Gln or Tyr; and
(k) a position corresponding to the position 537 of the amino acid sequence set forth in SEQ ID NO: 1 by substituting the amino acid residue Trp with Ala.

In a preferred embodiment, the mutant glucose oxidase of the invention has a reduced oxidase activity as compared to the wild-type glucose oxidase, and preferably has an increased dehydrogenase activity compared to the wild-type glucose oxidase. Preferably, the mutant glucose oxidase of the invention has an oxidase activity of 30% or less of that of the wild-type glucose oxidase, and preferably also has a dehydrogenase activity of 50% or more of the wild-type glucose oxidase. Also preferably, the mutant glucose oxidase of the invention has an increased dehydrogenase activity compared to the wild-type glucose oxidase.

In another aspect, the present invention provides an isolated polynucleotide encoding the mutant glucose oxidase of the present invention.

In yet another aspect, the present invention provides a vector comprising the polynucleotide of the invention.

In still another aspect, the present invention provides a host cell transformed with a vector of the invention.

In another aspect, the present invention provides a method for assaying glucose in a sample, comprising contacting the sample with the glucose oxidase of the invention and measuring the amount of the glucose oxidized by glucose oxidase.

In another aspect, the present invention provides a device for assaying glucose in a sample comprising the glucose oxidase of the invention and preferably an electron mediator.

In yet another aspect, the present invention provides a kit for assaying glucose in a sample comprising the glucose oxidase of the invention and preferably an electron mediator.

In another aspect, the present invention provides an enzyme electrode having the glucose oxidase of the invention which is immobilized on the electrode.

In another aspect, the present invention provides an enzyme sensor for assaying glucose comprising the enzyme electrode of the invention as a working electrode.

<u>Brief Description of Drawings</u>

Figure 1 shows the ratios of the oxidase (Ox) and dehydrogenase (Dh) activities of various glucose oxidase (GOx) mutants at a glucose substrate concentration of 100 mM to the wild-type activities. "n.d." in Fig. 1 and in the tables means "not detected".

Detailed Description of the Invention

The term "mutant" of a protein as used herein refers to a variant protein containing substitution in one or more of the amino acid residues on the protein at the indicated position(s). The term mutant is also used for a polynucleotide encoding such a mutant protein.

The phrase "a position corresponding to" as used herein means the position of an amino acid residue in a query amino acid sequence that is aligned with the amino acid residue in a reference amino acid sequence using a software AlignX of Vector NTI with default parameters (available from Invitrogen; see, Lu, G., and Moriyama, E. N. (2004) Vector NTI, a balanced all-in-one sequence analysis suite. Brief Bioinform 5, 378-88). Thus, "amino acid (AA) residue at a position corresponding to the position Y of the amino acid sequence set forth in SEQ ID NO: X" means the AA residue in a query amino acid sequence that is aligned with AA Y of SEQ ID NO: X when the query amino acid sequence is aligned with SEQ ID NO: X using AlignX of Vector NTI with default parameters. It should be noted that the AA Y of SEQ ID NO: X itself is also encompassed by this term.

The mutant glucose oxidase of the invention exhibit decreased oxidase activity while substantially retaining dehydrogenase activity.

As used herein "oxidase activity" is an enzymatic activity of the glucose oxidase to catalyze oxidation of glucose to generate gluconolacton with utilizing oxygen as an electron acceptor. The oxidase activity may be assayed by measuring the amount of generated H2O2 by any methods known in the art, for example, by reagents for H2O2 detection such as 4AA/TODB/POD (4-aminoantipyrine/N,N-Bis(4-sulfobutyl)-3-methylaniline disodium salt/horseradish peroxidase)or by Pt electrode. As used herein in the context of the relative or quantitative activity, the oxidase activity is specifically defined to be the mole amount of the substrate (glucose) oxidized per unit time measured by the amount of generated H2O2 at 25 °C in 10 mM PPB, pH 7.0, 1.5 mM TODB, 2 U/ml horseradish peroxidase (POD), and 1.5 mM 4-aminoanti-pyrine (4AA). The formation of quinoneimine dye may be measured spectrophotometrically at 546 nm.

As used herein, "dehydrogenase activity" is an enzymatic activity of the glucose oxidase to catalyze oxidation of glucose to generate gluconolacton with utilizing an electron mediator other than oxygen as an electron acceptor. The dehydrogenase activity may be assayed by measuring the amount of electron transferred to the mediator using, for example, mPMS/DCIP (1-methoxy-5-methylphenazinium methylsulfate/ 2,6-dichloroindophenol), cPES (trifluoro-acetate-1-(3-carboxy-propoxy)-5-ethyl-phenanzinium, NA BM31_1144 (N,N-bis-(hydroxyethyl)¬3-methoxy-nitrosoaniline hydrochloride, NA BM31_1008 (N,N-bis-hydroxy¬ethyl-4-nitrosoaniline) and N-N-4-dimethyl-nitrosoaniline.

As used herein in the context of the relative or quantitative activity, the dehydrogenase activity is specifically defined to be the mole amount of the substrate (glucose) oxidized per unit time measured by the amount of electron transferred to the mediator at 25 °C in 10 mM PPB (pH 7.0), 0.6 mM DCIP, and 6 mM methoxy PMS (mPMS).

The mutant glucose oxidase of the invention has a reduced oxidase activity as compared to the wild-type glucose oxidase, while substantially retaining the dehydrogenase activity.

Preferably, the mutant glucose oxidase of the invention has an oxidase activity of 50% or less of that of the wild-type glucose oxidase. More preferably, the mutant glucose oxidase of the invention has an oxidase activity of 40% or less, more preferably 30% or less, even more preferably 20% or less, most preferably 15% or less of that of the wild-type glucose oxidase. Also preferably, the mutant glucose oxidase of the invention has a dehydrogenase activity of 50% or more of the wild-type glucose oxidase. More preferably, the mutant glucose oxidase of the invention has a dehydrogenase activity of 70% or more, more preferably 90% or more, even more preferably 100% or more, most preferably more than 100% of the wild-type glucose oxidase.

In the wild-type glucose oxidase, the oxidase activity is about 3 to 4 times higher than the dehydrogenase activity. When dissolved oxygen is present in the assay system, the electron generated by the oxidation of the substrate will be preferentially transferred to the oxygen. Thus the enzyme activity measured in the presence of electron mediator will be greatly affected by the dissolved oxygen concentration. In contrast, the mutant glucose oxidase of the invention has the ratio of dehydrogenase/oxidase activity of about 2.0 or more, preferably 4.0 or more, more preferably 10 or more. Since the dehydrogenase activity exceed the oxidase activity, the enzyme activity of the glucose oxidase of the present invention will be less affected by the dissolved oxygen concentration, which is advantageous in utilizing the glucose oxidase in clinical diagnosis with a blood sample.

It should be understood that the numbering of the amino acid sequence in this application begins at the initial Met and that the claimed mutant glucose oxidase may or may not have the signal peptide.

In another aspect, the present invention provides an isolated polynucleotide encoding the mutant glucose oxidase of the present invention. The nucleotide sequence of polynucleotides coding for glucose oxidase may be easily obtained from public databases. The polynucleotide encoding the wild type glucose oxidase may be cloned from the genome of respective organisms using PCR or other known techniques. Mutations may be introduced by site-directed mutagenesis, PCR mutagenesis or any other techniques well known in the art. The amino acid residue to be mutated may be identified using any of software for sequence alignment available in the art. Alternatively, polynucleotide coding for the mutant glucose oxidase may be prepared by PCR using a series of chemically synthesized oligonucleotides, or fully synthesized.

The mutated glucose oxidase may be prepared by inserting the mutant gene into an appropriate expression vector and introducing the vector into an appropriate host cell, such as E. coli cells. The transformant is cultured and the glucose oxidase expressed in the transformant may be collected from the cells or culture medium. Alternatively, the recombinant glucose oxidase may be collected and refolded from the inclusion bodies prepared from the cells.

The recombinant glucose oxidase thus obtained may be purified by any of the purification techniques known in the art, including ion exchange column chromatography, affinity chromatography, liquid chromatography, filtration, ultrafiltration, salt precipitation, solvent precipitation, immunoprecipitation, gel electrophoresis, isoelectric electrophoresis and dialysis.

Thus, the present invention also encompasses a vector comprising the polynucleotide encoding the mutant glucose oxidase, a host cell transformed with such a vector, and a method for preparing the mutant glucose oxidase of the invention by culturing the transformant, collecting and purifying the mutant glucose oxidase from the culture.

The invention also encompasses a method for assaying glucose in a sample. The method comprises contacting the sample with the glucose oxidase of the invention and measuring the amount of the glucose oxidized by the glucose oxidase.

In another aspect, the present invention provides a device for assaying glucose in a sample comprising the glucose oxidase of the invention and an electron mediator.

The assay device may have a similar structure as any of conventional, commercially available amperometric biosensor test strips for monitoring the blood glucose level. One example of such a device has two electrodes (working electrode and reference or counter electrode) positioned on an insulating substrate, a reagent port and a sample receiver. The reagent port contains the mutated glucose oxidase of the invention and a mediator. When a sample such as blood sample is added to the sample receiver, glucose contained in the sample will react with glucose oxidase to generate current, which is indicative of the amount of glucose in the sample. Typical examples of electrochemical sensors suited for the determination of enzyme substrates are known, e.g. from WO 2004/113900 and US 5,997,817. As an alternative to electrochemical sensors, optical detection technologies might be used. Typically, such optical devices are based on color changes that occur in a reagent system comprising the enzyme, an electron mediator and an indicator. The color changes can be quantified using fluorescence, absorption or remission measurements. Typical examples of optical devices suited for the determination of enzyme substrates are known, e.g. from US 7,008,799, US 6,036,919, and US 5,334,508.

In yet another aspect, the present invention provides a kit for assaying glucose in a sample comprising the glucose oxidase of the invention and an electron mediator.

A kit for the measurement of glucose may be constructed using the enzyme of the present invention. In addition to the glucose oxidase of the invention, the kit contains buffer necessary for the measurement, appropriate mediator and, if necessary, enzymes such as peroxidase, standard solution of glucose for the preparation of a calibration curve and an instruction for use. The glucose oxidase of the present invention may be provided in various forms, for example, as a freeze-dried reagent or as a solution in an appropriate storage solution.

In another aspect, the present invention provides an enzyme electrode having the glucose oxidase of the invention which is immobilized on the electrode.

In another aspect, the present invention provides an enzyme sensor for assaying glucose comprising the enzyme electrode of the invention as a working electrode.

The concentration of the glucose in a sample may be determined by measuring the amount of electron generated by the enzyme reaction. Various sensor systems have been known in the art, including carbon electrode, metal electrode, and platinum electrode. The mutated glucose oxidase of the present invention is immobilized on the electrodes. Examples of the means for immobilization include cross-linking, encapsulating into a macromolecular matrix, coating with a dialysis membrane, optical cross-linking polymer, electroconductive polymer, oxidation-reduction polymer, and any combination thereof.

When measurement is conducted in an amperometric system using carbon electrode, gold electrode or platinum electrode provided with an immobilized enzyme is used as a working electrode, together with a counter electrode (such as platinum electrode) and a reference electrode (such as Ag/AgCl electrode). The electrodes are inserted into a buffer containing a mediator and kept at predetermined temperature. Predetermined voltage is applied to the working electrode, then a sample is added and increased value in electric current is measured. Examples of the mediator used in the assay include potassium ferricyanide, ferrocene, osmium derivative, ruthenium derivative, phenazine methosulfate, etc. It is generally also possible to use so-called two-electrode systems with one working electrode and one counter or pseudo-reference electrode.

Further, glucose may be assayed using a immobilized electron mediator in an amperometric system using carbon electrode, gold electrode, or platinum electrode. The enzyme is immobilized on the electrode together with an electron mediator such as potassium ferricyanide, ferrocene, osmium derivative, phenazine methosulfate in a macromolecular matrix by means of adsorption or covalent bond to prepare a working electrode. It is inserted into buffer together with a counter electrode (such as platinum electrode) and a reference electrode (such as Ag/AgCl electrode), and kept at a predetermined temperature. Predetermined voltage is applied to the working electrode, then the sample is added and increased value in electric current is measured.

Contents of all patents and reference documents cited in the present specification are entirely incorporated herein by reference.

Examples

The present invention will be illustrated in detail by way of the Examples below, although the present invention shall not be limited to those Examples.

**Example 1: Plasmid expressing GOx of *Penicillium amagasakiens***

pET22 gox WT was used as a plasmid expressing GOx of *Penicillium amagasakiens* (GenBank AADO1493). This plasmid has a DNA fragment containing the region of the GOx structural gene derived from *Penicillium amagasakiens* except for the signal sequence which is inserted in the *Nhe*I/*Eco*RI cloning site of a vector pET22. The GOx gene in this plasmid is controlled by a T7 promoter. The pET22 gox WT contains an ampicillin resistance gene.

**Example 2: Mutagenesis of GOx structural gene derived from *Penicillium* amagasakiens**

(1) Mutagenesis of residues 132 and 373

The *Penicillium amagasakiens*-derived GOx structural gene contained in the pET22 gox WT obtained in Example 1 was mutagenized such that serine at residue 132 and phenylalanine at residue 373 in GOx encoded by this gene were substituted by other amino acid residues.

Specifically, codon (TCC) for serine at residue 132 and codon (TTC) for phenylalanine at residue 373 in the GOx structural gene contained in the plasmid pET22 gox WT described in Example 1 were substituted by other amino acid codons using a commercially available site-directed mutagenesis kit (Stratagene Corp., QuikChange II Site-Directed Mutagenesis Kit).

The sequences of forward and reverse primers used in the amino acid residue substitution are shown in the tables below.

In notation representing a mutation, the number represents a position in the amino acid sequence containing the signal sequence of GOx; the alphabet described before the number represents an amino acid residue before amino acid substitution; and the alphabet described after the number represents an amino acid residue after amino acid substitution. For example, S 132A represents the substitution of serine at residue 132 to alanine.

In PCR reaction, a reaction solution of the composition shown below was subjected to reaction at 95°C for 30 seconds and then 15 repetitive cycles each involving 95°C for 30 seconds, 55°C for 1 minute, and 68°C for 8 minutes, followed by 68°C for 30 minutes and then kept at 4°C.

*Composition of reaction solution*

| | |
|---|---|
| Template DNA (5 ng/μL) | 2 μL |
| 10×reaction buffer | 5 μL |
| Forward primer (100 ng/μL) | 1.25 μL |
| Reverse primer (100 ng/μL) | 1.25 μL |
| dNTP | 1 μL |
| Distilled water | 38.5 μL |
| DNA polymerase | 1 μL |
| Total | 50 μL |

After the PCR reaction, 0.5 μL of *Dpn*I was added to the reaction solution and incubated at 37°C for 1 hour to degrade the template plasmid.

*Escherichia coli* DH5α (supE44, ΔlacU169 (ϕ801acZΔM15), hsdR17, recA1, endA1, gyrA96, thi-1, relA1) competent cells were transformed with the obtained reaction solution. From colonies grown on an LB agar medium (1% Bacto tryptone, 0.5% yeast extracts, 1% sodium chloride, 1.5% agar) containing ampicillin (50 μg/mL), plasmid DNA was prepared and sequenced to confirm that the mutation of interest was introduced in the GOx structural gene.

The plasmid confirmed to have the introduced mutation was digested with restriction enzymes NdeI and EcoRI to excise the mutagenized GOx structural gene, which was in turn inserted to a pET22 vector. DH5α was transformed with this plasmid, and a plasmid was extracted from the obtained colonies to obtain a GOx mutant expression plasmid.

**Table 1**

Primer for S132

| Amino acid substitution | Primer name | Sequence | SEQ ID NO. |
|---|---|---|---|
| S132A | S132AFw | 5'CTTGATAAACGGTGAC<u>GCG</u>TGGACTCGCCC 3' | 22 |
| S132A | S132ARv | 5'GGGCGAGTCCA<u>CGC</u>GTCACCGTTTATCAAG 3' | 23 |

**Table 2**

Primer for F373

| Amino acid substitution | Primer name | Sequence | SEQ ID NO. |
|---|---|---|---|
| F373A | F373AFw | 5'CAGGCCGTCTTC<u>GCG</u>GCCAATTTCACTGAG 3' | 24 |
| F373A | F373ARv | 5'CTCAGTGAAATTGGC<u>CGC</u>GAAGACGGCCTG 3' | 25 |
| F373L | F373LFw | 5'GTCAGGCCGTCTTC<u>CTG</u>GCCAATTTCACTGAG 3' | 26 |
| F373L | F373LRv | 5'CTCAGTGAAATTGGC<u>CAG</u>GAAGACGGCCTGAC 3' | 27 |
| F373P | F373PFw | 5'CAGGCCGTCTTC<u>CCG</u>GCCAATTTCACTGAG 3' | 28 |
| F373P | F373PRv | 5'CTCAGTGAAATTGGC<u>CGG</u>GAAGACGGCCTG 3' | 29 |
| F373W | F373WFw | 5'CAGGCCGTCTTC<u>TGG</u>GCCAATTTCACTGAG 3' | 30 |
| F373W | F373WRv | 5'CTCAGTGAAATTGGC<u>CCA</u>GAAGACGGCCTG 3' | 31 |
| F373Y | F373YFw | 5'CAGGCCGTCTTC<u>TAC</u>GCCAATTTCAC 3' | 32 |
| F373Y | F373YRv | 5'GTGAAATTGGC<u>GTA</u>GAAGACGGCCTG 3' | 33 |

**Example 3: Analysis of enzymatic activity of mutant GOx**

Mutant GOx was produced using the mutant GOx expression plasmid obtained in Example 2, and studied for its enzymatic activity.

(1) Culture

*Escherichia coli* strain BL21 (DE3) was transformed with the wild-type GOx expression plasmid prepared in Example 1 or the mutant GOx expression plasmid prepared in Example 2. These transformants were separately shake-cultured at 37°C for 12 hours in 3 mL of an LB medium (containing 100 μg/mL ampicillin) using an L-shaped tube. 1 mL each of these culture solutions was inoculated to a 500-mL Erlenmeyer flask with a baffle containing 100 mL of an LB medium (containing 100μ g/mL ampicillin) and gyratory-cultured at 37°C. At the point in time when OD600 reached around 0.6, IPTG (isopropyl-β-D-thiogalactopyranoside) was added thereto at a final concentration of 0.5 mM, followed by culture at 20°C for 24 hours.

(2) Preparation of inclusion body fraction

From the culture solution thus cultured, bacterial cells were collected and washed. Then, the obtained wet bacterial cells were suspended in a 20 mM potassium phosphate buffer (pH 6.8) and sonicated. Then, the homogenate was centrifuged at 17400xg at 4°C for 20 minutes, and the precipitate was used as an insoluble fraction. The obtained insoluble fraction was washed with a washing solution (1) (potassium phosphate buffer pH 6.8 + 100 mM NaCl + 1 mM EDTA + 1% Triton X-100) and centrifuged at 10000×g at 4°C for 10 minutes. The precipitate was washed with a washing solution (2) (potassium phosphate buffer pH 6.8 + 100 mM NaCl + 1 mM EDTA) and centrifuged at 10000×g at 4°C for 10 minutes. The precipitate was further washed with a washing solution (3) (2 M urea + 20 mM potassium phosphate buffer pH 6.8) and centrifuged at 10000×g at 4°C for 10 minutes. The inclusion body was collected as a precipitate. The most part of this inclusion body is GOx.

(3) Refolding of inclusion body

The inclusion body thus prepared was suspended in a solubilizing buffer (8 M urea + 30 mM dithiothreitol (DTT) + 20 mM potassium phosphate buffer pH 6.8), and this suspension was used as a solubilized inclusion body fraction. The solubilized inclusion body was diluted with a solubilizing buffer to a protein concentration of 0.1 mg/mL and dialyzed against a 100-fold volume or more of a refolding buffer (1 mM glutathione (reduced form) + 1 mM glutathione (oxidized form) + 0.05 mM flavin adenine dinucleotide + 10% (w/v) glycerol (vol/vol) + 20 mM potassium phosphate buffer pH 6.8) for 24 hours. Then, the resulting dialyzed solution was further dialyzed against 20 mM potassium phosphate buffer (pH 6.8) for 12 hours and centrifuged at 17400×g at 4°C for 3 minutes for removing protein aggregates. The supernatant was used as a GOx sample to determine glucose oxidase (GOx) and glucose dehydrogenase (GDH) activities at 25°C for each of wild-type GOx and mutant GOx.

(4) Determination of GOx activity

The GOx activity was determined by determining change in absorbance at 546 nm over time derived from a dye generated using peroxidase, a Trinder reagent (TODB), and 4-aminoantipyrine from hydrogen peroxide generated through reaction with the substrate. The reaction was performed under conditions shown below.

The reaction was initiated by adding the substrate to a reaction solution (10 mM potassium phosphate buffer pH 7.0 + 1.5 mM 4-aminoantipyrine + 1.5 mM TODB + 2 U/ml peroxidase; all the concentrations are final concentrations) containing the enzyme solution, and change in absorbance at 546 nm was determined. Various concentrations of glucose were used as the substrate. The amount of an enzyme that forms 1 μmol hydrogen peroxide for 1 minute is defined as 1 U. 38 mM-1 $cm^{-1}$ was used as the molar absorption coefficient of TODB at pH 7.0. The formula for calculating an activity value from change in absorbance is shown below.

$$U/ml = \Delta ABS_{546}/min \times 2/38 \times 10$$

$$U/mg = U/ml/protein\ mg/ml$$

(5) Determination of GDH activity

The GDH activity was determined by quantifying change in absorbance at 600 nm over time derived from the fading of DCIP reduced through reaction with the substrate. The reaction was performed under conditions shown below.

The reaction was initiated by adding the substrate to a reaction solution (10 mM potassium phosphate buffer pH 7.0 + 0.6 mM PMS + 0.06 mM DCIP; all the concentrations are final concentrations) containing the enzyme solution, and change in absorbance at 600 nm was determined. Those used in the GOx activity determination were used as the substrate. The amount of an enzyme that reduces 1 μmol DCIP is defined as 1 U. The activity value was calculated according to the formula shown below. 16.3 mM-1 $cm^{-1}$ was used as the molar absorption coefficient of DCIP at pH 7.0.

$$U/ml = \Delta ABS_{600}/min \times 1/16.3 \times 10$$

$$U/mg = U/ml/protein\ mg/ml$$

The results of activity determination of the wild-type GOx and the mutant GOx are shown in Tables 3 and 4 (different runs). Moreover, a graph showing the ratios of the oxidase and dehydrogenase activities of various mutants at a glucose substrate concentration of 10 mM to the wild-type activities is shown in FIG. 1. The S 132 and F373 mutants had reduced oxidase activity and improved dehydrogenase activity, compared with the wild-type. Among them, the S132A mutant had dehydrogenase activity improved to 4 times or more the wild-type and oxidase activity reduced to approximately 40% of the wide-type.

**Table 3**

| | GOx(U/mg) | | GDH(U/mg) | | GDH/GOx(%) | |
|---|---|---|---|---|---|---|
| | Substrate(mM) | | Substrate(mM) | | Substrate(mM) | |
| | 10 | 100 | 10 | 100 | 10 | 100 |
| WT | 9.05 (100%) | 15.0 (100%) | 2.25 (100%) | 3.83 (100%) | 24.8 | 25.6 |
| F373A | 1.38 (15.2%) | 1.53 (10.3%) | 2.68 (119%) | 3.38 (88.2%) | 194 | 220 |
| F373L | 7.62 (84.2%) | 10.0 (67.1%) | 4.38 (195%) | 6.27 (164%) | 57.5 | 62.4 |
| F373W | 2.91 (32.2%) | - | 0.496 (22.0%) | - | 17.0 | - |
| F373Y | 6.94 (76.7%) | 10.2 (68.0%) | 1.35 (60.0%) | 2.03 (53.0%) | 19.4 | 20.0 |

**Table 4**

| | GOx(U/mg) | | GDH(U/mg) | | GDH/GOx(%) | |
|---|---|---|---|---|---|---|
| | Substrate(mM) | | Substrate(mM) | | Substrate(mM) | |
| | 10 | 100 | 10 | 100 | 10 | 100 |
| WT | 31.3 (100%) | 47.4 (100%) | 8.13 (100%) | 13.1 (100%) | 26 | 27.7 |
| S132A | 10.8 (34.50%) | 14.9 (31.40%) | 24.3 (299%) | 53.5 (408%) | 225 | 360 |
| S132C | 0.592 (1.89%) | 1.86 (3.92%) | 0.779 (9.58%) | 4.21 (32.10%) | 132 | 226 |
| S132I | 0.171 (0.55%) | 1.16 (2.46%) | 0.319 (3.92%) | 2.74 (20.90%) | 186 | 236 |
| S132N | 0.0098 (0.03%) | 0.0514 (0.11%) | 0.0169 (0.21%) | 0.185 (1.41% | 173 | 360 |
| S132R | n.d. | n.d. | n.d. | n.d. | - | - |
| S132D | n.d. | n.d. | n.d. | n.d. | - | - |
| S132T | 6.94 (29.10%) | 11.2 (28.90%) | 1.84 (27.70%) | 3.02 (26.90%) | 26.5 | 27.1 |
| S132V | 0.0858 (0.36%) | 0.531 (1.38%) | 0.298 (4.49%) | 1.37 (12.20%) | 347 | 259 |
| S132Y | n.d. | n.d. | n.d. | n.d. | - | - |
| S132E | n.d. | n.d. | n.d. | n.d. | - | - |
| S132H | n.d. | n.d. | n.d. | n.d. | - | - |
| S132M | n.d. | 0.0265 (0.06%) | n.d. | 0.101 (0.84%) | - | 380 |
| S132Q | n.d. | 0.0399 (0.08%) | n.d. | 0.17 (1.42%) | - | 426 |
| S132A/F373A | 0.358 (3.96%) | 0.434 (2.90%) | 3.55 (158%) | 8.07 (211%) | 991 | $1.86 \times 10^3$ |
| S132A/F373L | 2.65 (29.30%) | 3.2 (21.40%) | 8.75 (389%) | 19.7 (515%) | 330 | 616 |

**Example 4: Preparation and analysis of enzymatic activity of wild type and mutant GOx derived from *Apergillus niger***

Wild type and mutant GOx from *A. niger* (SwissProt P13006) were prepared in the same manner as described in Examples 1 and 2. The threonine residue at 132 and phenylalanine reside at 373 of *A. niger* (SwissProt P13006) correspond to serine residue at 132 and phenylalanine residue at 373 of *Penicillium amagasakiens* (GenBank AADO1493), respectively.

Mutant GOx from *A. niger* was prepared and its enzymatic activity was analyzed in the same manner as described in Example 3. The results from wild type and mutant GOxs are summarized in Table 5 below.

**Table 5**

| | GOx(U/mg) | | GDH(U/mg) | | GDH/GOx(%) | |
|---|---|---|---|---|---|---|
| | Substrate(mM) | | Substrate(mM) | | Substrate(mM) | |
| | 50 mM | 200 mM | 50 mM | 200 mM | 50 mM | 200 mM |
| WT | 3.43 (100%) | 5.01 (100%) | 1.37 (100%) | 2 (100%) | 40 (100%) | 40 (100%) |
| T132A | 2.46 (71.70%) | 3.82 (76.20%) | 4.04 (295%) | 10.1 (502%) | 164 (411%) | 263 (658%) |
| T132S | 6.64 (193%) | 10.2 (204%) | 4.43 (322%) | 7.13 (356%) | 66.7 (167%) | 69.7 (174%) |
| F373Y | 2.05 (59.80%) | 3.14 (62.80%) | 1.16 (84.10%) | 2.08 (104%) | 56.3 (141%) | 66.2 (165%) |

Table 6 and 7 show alignment of the amino acid sequences that are annotated to be glucose oxidases. The entire sequences of these mutated glucose oxidases are set forth in SEQ ID NOs: 1-21. Alignment was created using AlignX application of Vector NTI suite 6.0. Those skilled in the art will appreciate that another alignment software programs such as Blast will provide the same or substantially the same alignment.

It is evident from Table 6 that Ser132 of SEQ ID NO:1 is conserved among the amino acid sequences listed in Table 6. Accordingly, a person skilled in the art can easily identify the Ser or Thr residue corresponding to the Ser132 of SEQ ID NO:1 within the conserved region using any of commercially available software programs for sequence alignment, and understand that a mutant glucose oxidase is easily prepared by introducing modification on that Ser or Thr residue. (Please note: * Databases: gb: GenBank; sp: Swissprot; ref: RefSeq; emb: EMBL; pdb: Protein Data Bank; ** SEQ ID NOs represent the full length sequence)

**Table 6**

| origin* | position of mutation | | | | SEQ ID NO** |
|---|---|---|---|---|---|
| gb\|AAD01493 | S132 | 122 | LGGSTLINGD**S**WTRPDKVQID | 142 | 1 |
| gb\|ABM63225 | S132 | 122 | LGGSTLINGD**S**WTRPDKVQID | 142 | 2 |
| sp\|Q92452 | S132 | 122 | LGGSTLINGD**S**WTRPDKVQID | 142 | 3 |
| ref\|XP_002482295 | S163 | 153 | LGGSTLINGD**S**WTRPDKIQID | 173 | 4 |
| emb\|CAE47418 | S132 | 122 | LGGSTLINGD**S**WTRPDKVQID | 142 | 5 |
| ref\|XP_002563451 | S132 | 122 | LGGSTLINGD**S**WTRPDKVQID | 142 | 6 |
| ref\|XP_001217613 | S156 | 146 | LGGSTLINGD**S**WTRPDKVQID | 166 | 7 |
| ref\|XP_001215424 | S130 | 120 | LGGSTLINGD**S**WTRPDKVQID | 140 | 8 |
| ref\|XP_001727544 | S133 | 123 | LGGSTLINGG**S**WTRPDKVQID | 143 | 9 |
| ref\|XP_002375824 | S133 | 123 | LGGSTLINGG**S**WTRPDKVQID | 143 | 10 |
| ref\|XP_001216461 | S133 | 123 | LGGSTLVNGG**S**WTSPDKVQLD | 143 | 11 |
| gb\|ADP03053 | T110 | 100 | LGGSTLVNGG**T**WTRPHKAQVD | 120 | 12 |
| gb\|ACR56326 | T132 | 122 | LGGSTLVNGG**T**WTRPHKAQVD | 142 | 13 |
| sp\|P13006 | T132 | 122 | LGGSTLVNGG**T**WTRPHKAQVD | 142 | 14 |
| gb\|ABG54443 | T108 | 98 | LGGSTLVNGG**T**WTRPHKAQVD | 118 | 15 |
| gb\|AAV68194 | T108 | 98 | LGGSTLVNGG**T**WTRPHKAQVD | 118 | 16 |
| gb\|AAF59929 | T132 | 122 | LGGSTLVNGG**T**WTRPHKAQVD | 142 | 17 |
| gb\|ABG66642 | T108 | 98 | LGGSSLVNGG**T**WTRPHKAQVD | 118 | 18 |
| emb\|CAC12802 | T131 | 121 | LGGSTLVNGG**T**WTRPHKVQVD | 141 | 19 |
| ref\|XP_001588502 | T130 | 120 | LGGSTLINGA**T**WTRPHKIQVD | 140 | 20 |
| ref\|XP_001395420 | T163 | 153 | LGGSTLINGG**T**WTRPHKSQLD | 173 | 21 |

It is evident from Table 7 that Phe373 of SEQ ID NO:1 is conserved among the amino acid sequences listed in Table 7. Accordingly, a person skilled in the art can easily identify the Phe residue corresponding to the Phe373 of SEQ ID NO:1 within the conserved region using any of commercially available software programs for sequence alignment, and understand that a mutant glucose oxidase is easily prepared by introducing modification on that Phe residue. (Please note: * Databases: gb: GenBank; sp: Swissprot; ref: RefSeq; emb: EMBL; pdb: Protein Data Bank; ** SEQ ID NOs represent the full length sequence).

**Table 7**

| origin* | position of mutation | | | | SEQ ID NO** |
|---|---|---|---|---|---|
| gb\|AAD01493 | F373 | 363 | AGAGQGQ--AVFFANFTETFGDY | 383 | 1 |
| gb\|ABM63225 | F373 | 363 | AGAGQGQ--AVFFANFTETFGDY | 383 | 2 |
| sp\|Q92452 | F373 | 363 | AGAGQGO--AVFFANFTETFGDY | 383 | 3 |
| ref\|XP_002482295 | F404 | 394 | AGAGQGQ--AVFFANFTETFGDY | 414 | 4 |
| emb\|CAE47418 | F373 | 363 | AGTGQGQ--AVFFANFTEVFGDY | 383 | 5 |
| ref\|XP_002563451 | F372 | 363 | A-PGQGQ--AAYFANFTEVLGDH | 382 | 6 |
| ref\|XP_001217613 | F397 | 387 | AGFGQGQ--AVYFANFTETFGED | 407 | 7 |
| ref\|XP_001215424 | F371 | 361 | AGFGQGQ--AVYFANFTETFEED | 381 | 8 |
| ref\|XP_001727544 | F374 | 364 | SGAGQGQ--AVYFASFNETFGDY | 384 | 9 |
| ref\|XP_002375824 | F374 | 364 | SGAGQGQ--AVYFASFNETFGDY | 384 | 10 |
| ref\|XP_001216461 | F374 | 364 | TGAGQGQ--AVYFANFTETFGDH | 384 | 11 |
| gb\|ADP03053 | F351 | 341 | AGAGQGQ--AAWFATFNETFGDY | 361 | 12 |
| gb\|ACR56326 | F373 | 363 | AGAGQGQ--AAWFATFNETFGDY | 383 | 13 |
| sp\|P13006 | F373 | 363 | AGAGQGQ--AAWFATFNETFGDY | 383 | 14 |
| gb\|ABG54443 | F349 | 339 | AGAGQGQ--AAWFATFNETFGDY | 359 | 15 |
| gb\|AAV68194 | F349 | 339 | AGAGQGQ--AAWFATFNETFGDY | 359 | 16 |
| gb\|AAF59929 | F373 | 363 | AGAGQGQ--AAWFATFNETFGDY | 383 | 17 |
| gb\|ABG66642 | F349 | 339 | AGAGQGQ--AAWFATFNETLGDY | 359 | 18 |
| emb\|CAC12802 | F372 | 362 | AGAGQGQ--VAIFATFNETFGDY | 382 | 19 |
| ref\|XP_001588502 | F373 | 363 | LGYGQGQ--AIYFATFNETFGKY | 383 | 20 |
| ref_XP_001395420 | F422 | 412 | EANGQGQ--AAYFATFAEIFGKD | 432 | 21 |

**Example 5: Additional GOx mutants**

Additional GOx mutants derived from GOx of Penicillium amagasakiens (GenBank AADO1493) and Aspergillus niger (SwissProt P13006) were prepared and their enzyme activity was analyzed in the same manner as described in Examples 1-4. The ratio of glucose dehydrogenase activity:glucose oxidase activity of each mutant are summarized in Tables 8a, 8b, 9a and 9b below, with the ratio of the wild-type glucose oxidase being 100%. The SEQ ID NOs of the amino acid sequences of each wild-type GOx are shown in Tables 6 and 7. Also the alignment of the amino acid sequences around the mutated positions are shown in Tables 10a-h.

Table 8a

| 1GPE (AADO1493) | | | |
|---|---|---|---|
| | Type of enzyme | Dh/Ox Ratio vs wild type | |
| | | 10mM Glucose | 100mM Glucose |
| | Wild type | 100% | 100% |

(continued)

| 1GPE (AADO1493) | | | |
|---|---|---|---|
| | Type of enzyme | Dh/Ox Ratio vs wild type | |
| | | 10mM Glucose | 100mM Glucose |
| Thr53 | Thr53Ser | 125% | - |
| Ilel16 | Ile116Val | 101% | - |
| Ser132 | Ser132Ala<br>Ser132Thr<br>Ser132val<br>Ser132Cys<br>Ser132Ile | 839%<br>95%<br>1248%<br>508%<br>715% | 1235%<br>93%<br>888%<br>817%<br>851% |
| Ile237 | Ile237Val | - | 182% |
| Val371 | Vla1371Thr<br>Val371Ala | 118%<br>111% | 113%<br>107% |
| Phe373 | Phe373Leu<br>Phe373Tyr<br>Phe373Ala | 246%<br>171%<br>862% | 231%<br>165%<br>885% |
| Glu434 | Glu434Gln | 215% | 134% |
| Phe436 | Phe436Trp<br>Phe436Ala<br>Phe436Leu<br>Phe436Tyr | -<br>544%<br>-<br>- | 1365%<br>592%<br>127%<br>783% |
| Trp448 | Trp448Ala<br>Trp448Ile<br>Trp448Ser | 1630%-<br>-<br>- | 1440%<br>4382%<br>644% |
| Trp537 | Trp537Ala | 546% | 550% |

Table 8b

| **1GPE (AADO1493)** | | | |
|---|---|---|---|
| | Type of enzyme | Dh/Ox Ratio vs wild type | |
| | | 10mM Glucose | 100mM Glucose |
| | Wild type | 100% | 100% |
| Phe373 | Phe373Met<br>Phe373Trp | -<br>- | 195%<br>111% |
| Phe436 | Phe436Met<br>Phe436Glu | -<br>- | 155<br><no oxidase> |
| Trp448 | Trp448Val<br>Trp448Met<br>Trp448Thr<br>Trp448Cys | -<br>-<br>-<br>- | 4011%<br><no oxidase><br>2290%<br>4427% |

Table 9a

| 1CF3 (P13006 | | | |
|---|---|---|---|
| | Type of enzyme | Dh/Ox Ratio vs wild type | |
| | | 50mM Glucose | 200mM Glucose |
| | Wild type | 100% | 100% |
| Thr132 | Thr132Ala<br>Thr132Ser<br>Thr132Val<br>Thr132Trp<br>Thr132Cys | 403%<br>155%<br>323%<br>545%<br>1013% | 537%<br>112%<br>503%<br>416%<br>875% |
| Thr134 | Thr134Ala | 373% | 445% |
| Phe237 | Phe237Ile | 142% | 120% |
| Ala371 | Ala371Val | 223% | 180% |
| Phe373 | Phe373Leu<br>Phe373Tyr<br>Phe373Met<br>Phe373Asn | 293%<br>170%<br>149%<br>235% | 316%<br>159%<br>141%<br>206% |
| Trp448 | Trp448Ala | 488% | 610% |
| double mutant | Thr132Ala/Phe373Tyr | 928% | 1511% |
| double mutant | Thr132Ser/Phe373Leu | 633% | 794% |

Table 9b

| 1CF3 (P13006 | | | |
|---|---|---|---|
| | Type of enzyme | Dh/Ox Ratio vs wild type | |
| | | 50mM Glucose | 200mM Glucose |
| | Wild type | 100% | 100% |
| Thr134 | Thr134Ile<br>Thr134Met | -<br>- | 300%<br>170% |
| Phe237 | Pre237Ala<br>Phe237Val<br>Phe237Met<br>Phe237Ser<br>Phe237Asp<br>Phe237Leu<br>Phe237Thr<br>Phe237Asn<br>Phe237Arg<br>Phe237Cys | <br>-<br>-<br>-<br>-<br>-<br>-<br>-<br>-<br>- | <no oxidase><br>210%<br>280%<br>780%<br>1600%<br>170%<br>2900%<br>600%<br><no oxidase><br>650% |
| Phe436 | Phe436Leu<br>Phe436Ile<br>Phe436Met | -<br>-<br>- | 1760%<br>1818%<br>140% |
| Trp448 | Trp448Gly<br>Trp448Val<br>Trp448Leu<br>Trp448Ser | -<br>-<br>-<br>- | 819%<br>11134%<br>7742%<br>426% |

(continued)

| 1CF3 (P13006 | | | |
|---|---|---|---|
| | Type of enzyme | Dh/Ox Ratio vs wild type | |
| | | 50mM Glucose | 200mM Glucose |
| | Trp448Asn | - | 642% |
| | Trp448Asp | - | 4368% |
| | Trp448Lys | - | <no oxidase> |
| | Trp448Ile | - | 940% |
| | Trp448Met | - | 1100% |
| | Trp448Phe | - | 400% |
| | Trp448Thr | - | 310% |
| | Trp448Cys | - | 590% |
| | Trp448Gln | - | 680% |
| | Trp448Tyr | - | 270% |

Table 10a. Amino acid sequences around Thr535

| origin | position of mutation | amino acid sequences around mutated position(s) | | |
|---|---|---|---|---|
| gbAAD01493 | T53 | 43 | YDYIIAGGGL**T**GLTVAAKLTE | 63 |
| gbABM63225 | T53 | 43 | YDYIIAGGGL**T**GLTVAAKLTE | 63 |
| spQ92452 | T53 | 43 | YDYIIAGGGL**T**GLTVAAKLTE | 63 |
| refXP_002482295 | T84 | 74 | YDYIIAGGGL**T**GLTVAAKLTE | 94 |
| embCAE47418 | T53 | 43 | YDYIIAGGGL**T**GLTVAAKLSE | 63 |
| refXP_002563451 | T53 | 43 | YDYVIAGGGL**T**GLTVAAKLSE | 63 |
| refXP_001217613 | T77 | 67 | FDYIIAGGGL**T**GLTVAAKLTE | 87 |
| refXP_001215424 | T51 | 41 | FDYIIAGGGL**T**GLTVAAKLTE | 61 |
| refXP_001727544 | T53 | 43 | FDYVIAGGGL**T**GLTVATKLTE | 63 |
| refXP_002375824 | T53 | 43 | FDYVIAGGGL**T**GLTVATKLTE | 63 |
| refXP_001216461 | T53 | 43 | VDYIIAGGGL**T**GLTVAAKLTE | 63 |
| gbADP03053 | T30 | 20 | VDYIIAGGGL**T**GLTTAARLTE | 40 |
| gbACR56326 | T52 | 42 | VDYIIAGGGL**T**GLTTAARLTE | 62 |
| spP13006 | T52 | 42 | VDYIIAGGGL**T**GLTTAARLTE | 62 |
| gbABG54443 | T28 | 18 | VDYIIAGGGL**T**GLTTAARLTE | 38 |
| gbAAV68194 | T28 | 18 | VDYIIAGGGL**T**GLTTAARLTE | 38 |
| gbAAF59929 | T52 | 42 | VDYIIAGGGL**T**GLTTAARLTE | 62 |
| gbABG66642 | T28 | 18 | VDYIIAGGGL**T**GLTTAARLTE | 38 |
| embCAC12802 | T51 | 41 | VDDIIAGGGL**T**GLTTAARLTE | 61 |
| refXP_001588502 | T50 | 40 | FDYIVAGGGL**T**GLTAAAILSK | 60 |

Table 10b. Amino acid sequences around Ile116

| origin | position of mutation | amino acid sequences around mutated position(s) | | |
|---|---|---|---|---|
| gbAAD01493 | I116 | 106 | VPL--INNRTNN**I**KAGKGLGGST | 126 |

(continued)

| origin | position of mutation | amino acid sequences around mutated position(s) | | |
|---|---|---|---|---|
| gbABM63225 | I116 | 106 | VPL--INNRTNN**I**KAGKGLGGST | 126 |
| spQ92452 | I116 | 106 | VPL--INNRTNN**I**KAGKGLGGST | 126 |
| refXP_002482295 | I147 | 137 | VPL--INNRTNN**I**KAGKGLGGST | 157 |
| embCAE47418 | I116 | 106 | VPL--INNRTSS**I**KSGKGLGGST | 126 |
| refXP_002563451 | I116 | 106 | VPL--INNRTGE**I**KSGLGLGGST | 126 |
| refXP_001217613 | I140 | 130 | VPL--INNRTDN**I**KSGKGLGGST | 150 |
| refXP_001215424 | I114 | 104 | VPL--INNRTDN**I**KSGKGLGGST | 124 |
| refXP_001727544 | I117 | 106 | VPLA-VNNRTEL**I**RSGNGLGGST | 127 |
| refXP_002375824 | I117 | 106 | VPLA-VNNRTEL**I**RSGNGLGGST | 127 |
| refXP_001216461 | I117 | 106 | VPMG-INNRTLD**I**KSGKGLGGST | 127 |
| gbADP03053 | I94 | 83 | VELA-TNNQTAL**I**RSGNGLGGST | 104 |
| gbACR56326 | I116 | 105 | VELA-TNNQTAL**I**RSGNGLGGST | 126 |
| spP13006 | I116 | 105 | VELA-TNNQTAL**I**RSGNGLGGST | 126 |
| gbABG54443 | I92 | 81 | VELA-TNNQTAL**I**RSGNGLGGST | 102 |
| gbAAV68194 | I92 | 81 | VELA-TNNQTAL**I**RSGNGLGGST | 102 |
| gbAAF59929 | I116 | 105 | VELA-TNNQTAL**I**RSGNGLGGST | 126 |
| gbABG66642 | I92 | 81 | VELA-TNNQTAL**I**RSGNGLGGSS | 102 |
| embCAC12802 | I115 | 104 | VELA-TNNLTEL**I**RSGNGLGGST | 125 |
| refXP_001588502 | I114 | 103 | LNQT-ADIPQQT**I**RSGRGLGGST | 124 |

Table 10c. Amino acid sequences around S/T132, W133 and T134

| origin | position of mutation | amino acid sequences around mutated position(s) | | |
|---|---|---|---|---|
| gbAAD01493 | S132,W133,T134 | 122 | LGGSTLINGD**SWT**RPDKVQID | 142 |
| gbABM63225 | S132,W133,T134 | 122 | LGGSTLINGD**SWT**RPDKVQID | 142 |
| spQ92452 | S132,W133,T134 | 122 | LGGSTLINGD**SWT**RPDKVQID | 142 |
| refXP_002482295 | S163,W164,T165 | 153 | LGGSTLINGD**SWT**RPDKIQID | 173 |
| embCAE47418 | S132,W133,T134 | 122 | LGGSTLINGD**SWT**RPDKVQID | 142 |
| refXP_002563451 | S132,W133,T134 | 122 | LGGSTLINGD**SWT**RPDKVQID | 142 |
| refXP_001217613 | S156,W157,T158 | 146 | LGGSTLINGD**SWT**RPDKVQID | 166 |
| refXP_001215424 | S130,W131,T132 | 120 | LGGSTLINGD**SWT**RPDKVQID | 140 |
| refXP_001727544 | S133,W134,T135 | 123 | LGGSTLINGG**SWT**RPDKVQID | 143 |
| refXP_002375824 | S133,W134,T135 | 123 | LGGSTLINGG**SWT**RPDKVQID | 143 |
| refXP_001216461 | S133,W134,T135 | 123 | LGGSTLVNGG**SWT**SPDKVQLD | 143 |
| gbADP03053 | T110,W111,T112 | 100 | LGGSTLVNGG**TWT**RPHKAQVD | 120 |
| gbACR56326 | T132,W133,T134 | 122 | LGGSTLVNGG**TWT**RPHKAQVD | 142 |
| spP13006 | T132,W133,T134 | 122 | LGGSTLVNGG**TWT**RPHKAQVD | 142 |
| gbABG54443 | T108,W109,T110 | 98 | LGGSTLVNGG**TWT**RPHKAQVD | 118 |

(continued)

| origin | position of mutation | amino acid sequences around mutated position(s) | | |
|---|---|---|---|---|
| gbAAV68194 | T108,W109,T110 | 98 | LGGSTLVNGG**TWT**RPHKAQVD | 118 |
| gbAAF59929 | T132,W133,T134 | 122 | LGGSTLVNGG**TWT**RPHKAQVD | 142 |
| gbABG66642 | T108,W109,T110 | 98 | LGGSSLVNGG**TWT**RPHKAQVD | 118 |
| embCAC12802 | T131,W132,T133 | 121 | LGGSTLVNGG**TWT**RPHKVQVD | 141 |
| refXP_001588502 | T130,W131,T132 | 120 | LGGSTLINGA**TWT**RPHKIQVD | 140 |

Table 10d. Amino acid sequences around I/F237

| origin | position of mutation | amino acid sequences around mutated position(s) | | |
|---|---|---|---|---|
| gbAAD01493 | I237 | 227 | LCGHPRGVSM**I**MNNLDE--NQVR | 247 |
| gbABM63225 | I237 | 227 | LCGHPRGVSM**I**MNNLDE--NQVR | 247 |
| spQ92452 | I237 | 227 | LCGHPRGVSM**I**MNNVDE--NQVR | 247 |
| refXP_002482295 | I268 | 258 | LCGHPRGVSM**I**MNNVDE--NQVR | 278 |
| embCAE47418 | I237 | 227 | LCGHPRGVSM**I**YNNLDE--NQVR | 247 |
| refXP_002563451 | I237 | 227 | HCGHPRGVSM**I**PNNLHE--NQIR | 247 |
| refXP_001217613 | I261 | 251 | HCGHPRGVSM**I**PNNLLE--DQVR | 271 |
| refXP_001215424 | I235 | 225 | HCGHPRGVSM**I**PNNLLE--DQVR | 245 |
| refXP_001727544 | I238 | 228 | HCGHPRGVSM**I**PNAVHE--DQTR | 248 |
| refXP_002375824 | I238 | 228 | HCGHPRGVSM**I**PNAVHE--DQTR | 248 |
| refXP_001216461 | I238 | 228 | HCGHPRGVSM**I**LNSLHE--DQTR | 248 |
| gbADP03053 | F215 | 205 | GCGDPHGVSM**F**PNTLHE--DQVR | 225 |
| gbACR56326 | F237 | 227 | GCGDPHGVSM**F**PNTLHE--DQVR | 247 |
| spP13006 | F237 | 227 | GCGDPHGVSM**F**PNTLHE--DQVR | 247 |
| gbABG54443 | F213 | 203 | GCGDPHGVSM**F**PNTLHE--DQVR | 223 |
| gbAAV68194 | F213 | 203 | GCGDPHGVSM**F**PNTLHE--DQVR | 223 |
| gbAAF59929 | F237 | 227 | GCGDPHGVSM**F**PNTLHE--DQVR | 247 |
| gbABG66642 | F213 | 203 | GCGDPHGVSM**F**PNTLHE--DQVR | 223 |
| embCAC12802 | F236 | 226 | GCGDPHGVSM**F**PNTLHE--DQVR | 246 |
| refXP_001588502 | F235 | 225 | SCGNPHGVSM**F**PNSLHANWNQTR | 247 |

Table 10e. Amino acid sequences around V/A/I371 and F373

| origin | position of mutation | amino acid sequences around mutated position(s) | | |
|---|---|---|---|---|
| gbAAD01493 | V371,F373 | 363 | AGAGQGQ--A**V**F**F**ANFTETFGDY | 383 |
| gbABM63225 | V371,F373 | 363 | AGAGQGQ--A**V**F**F**ANFTETFGDY | 383 |
| spQ92452 | V371,F373 | 363 | AGAGQGQ--A**V**F**F**ANFTETFGDY | 383 |
| refXP_002482295 | V402,F404 | 394 | AGAGQGQ--A**V**F**F**ANFTETFGDY | 414 |
| embCAE47418 | V371,F373 | 363 | AGTGQGQ--A**V**F**F**ANFTEVFGDY | 383 |
| refXP_002563451 | A370,F372 | 363 | A-PGQGQ--A**A**Y**F**ANFTEVLGDH | 382 |

(continued)

| origin | position of mutation | amino acid sequences around mutated position(s) | | |
|---|---|---|---|---|
| refXP_001217613 | V395,F397 | 387 | AGFGQGQ--AB**V**Y**F**ANFTETFGED | 407 |
| refXP_001215424 | V369,F371 | 361 | AGFGQGQ--A**V**Y**F**ANFTETFEED | 381 |
| refXP_001727544 | V372,F374 | 364 | SGAGQGQ--A**V**Y**F**ASFNETFGDY | 384 |
| refXP_002375824 | V372,F374 | 364 | SGAGQGQ--A**V**Y**F**ASFNETFGDY | 384 |
| refXP_001216461 | V372,F374 | 364 | TGAGQGQ--A**V**Y**F**ANFTETFGDH | 384 |
| gbADP03053 | A349,F351 | 341 | AGAGQGQ--A**A**W**F**ATFNETFGDY | 361 |
| gbACR56326 | A371,F373 | 363 | AGAGQGQ--A**A**W**F**ATFNETFGDY | 383 |
| spP13006 | A371,F373 | 363 | AGAGQGQ--A**A**W**F**ATFNETFGDY | 383 |
| gbABG54443 | A347,F349 | 339 | AGAGQGQ--A**A**W**F**ATFNETFGDY | 359 |
| gbAAV68194 | A347,F349 | 339 | AGAGQGQ--A**A**W**F**ATFNETFGDY | 359 |
| gbAAF59929 | A371,F373 | 363 | AGAGQGQ--A**A**W**F**ATFNETFGDY | 383 |
| gbABG66642 | A347,F349 | 339 | AGAGQGQ--A**A**W**F**ATFNETLGDY | 359 |
| embCAC12802 | A370,F372 | 362 | AGAGQGQ--V**A**I**F**ATFNETFGDY | 382 |
| refXP_001588502 | 1371,F373 | 363 | LGYGQGQ--A**I**Y**F**ATFNETFGKY | 383 |

Table 10f. Amino acid sequences around E434 and F436

| origin | position of mutation | amino acid sequences around mutated position(s) | | |
|---|---|---|---|---|
| gbAAD01493 | E434,F436 | 426 | LDED--VAFA**E**L**F**MDT--EGKINFD | 446 |
| gbABM63225 | E434,F436 | 426 | LDED--VAFA**E**L**F**MDT--EGKINFD | 446 |
| spQ92452 | E434,F436 | 426 | LDED--VAFA**E**L**F**MDT--EGKINFD | 446 |
| refXP_002482295 | E435,F467 | 457 | LDED--VAFA**E**L**F**MDT--EGKINFD | 477 |
| embCAE47418 | E434,F436 | 426 | LEED--VAYA**E**L**F**MDT--SGKINFD | 446 |
| refXP_002563451 | E433,F435 | 425 | LDED--VAFA**E**L**F**FDT--EGKINFD | 445 |
| refXP_001217613 | E458,F460 | 450 | LNED--VAYA**E**L**F**LDT--SGQINFD | 470 |
| refXP_001215424 | E432,F434 | 424 | LNED--VAYA**E**L**F**LDT--SGQINFD | 444 |
| refXP_001727544 | E435,F437 | 427 | LNED--VAFA**E**L**F**LDT--EGKINFD | 447 |
| refXP_002375824 | E435,F437 | 427 | LNED--VAFA**E**L**F**LDT--EGKINFD | 447 |
| refXP_001216461 | E435,F437 | 427 | LEDD--VAFV**E**F**F**FDS--NGMINFD | 447 |
| gbADP03053 | E412,F414 | 404 | VNHN--VAYS**E**L**F**LDT--AGVASFD | 424 |
| gbACR56326 | E434,F436 | 426 | VKDN--VAYS**E**L**F**LDT--AGVASFD | 446 |
| spP13006 | E434,F436 | 426 | VNHN--VAYS**E**L**F**LDT--AGVASFD | 446 |
| gbABG54443 | E410,F412 | 402 | VKDN--VAYS**E**L**F**LDT--AGVASFD | 422 |
| gbAAV68194 | E410,F412 | 402 | VKDN--VAYS**E**L**F**LDT--AGVASFD | 422 |
| gbAAF59929 | E434,F436 | 426 | VNHN--VAYS**E**L**F**LDT--AGVASFD | 426 |
| gbABG66642 | E410,F412 | 402 | VKDN--VAYS**E**L**F**LDT--AGVASFG | 422 |
| embCAC12802 | E433,F435 | 425 | VNHN--VAYS**E**L**F**LDT--AGAVSFT | 445 |
| refXP_001588502 | E434,F436 | 426 | TKDN--IAYS**E**L**F**MDT--EGAINFD | 446 |

Table 10g. Amino acid sequences around W448

| origin | position of mutation | amino acid sequences around mutated position(s) | | |
|---|---|---|---|---|
| gbAAD01493 | W448 | 438 | DT--EGKINFDL**W**DLIPFTRGSV | 458 |
| gbABM63225 | W448 | 438 | DT--EGKINFDL**W**DLIPFTRGSV | 458 |
| spQ92452 | W448 | 438 | DT--EGKINFDL**W**DLIPFTRGSV | 458 |
| refXP_002482295 | W479 | 469 | DT--EGKINFDL**W**DLIPFTRGSV | 489 |
| embCAE47418 | W448 | 438 | DT--SGKINFDL**W**DLIPFTRGSV | 458 |
| refXP_002563451 | W447 | 437 | DT--EGKINFDI**W**NLIPFTRGSV | 457 |
| refXP_001217613 | W472 | 462 | DT--SGQINFDL**W**DLIPFTRGST | 482 |
| refXP_001215424 | W446 | 436 | DT--SGQINFDL**W**DLIPFTRGST | 456 |
| refXP_001727544 | W449 | 439 | DT--EGKINFDL**W**DLIPFTRGSV | 459 |
| refXP_002375824 | W449 | 439 | DT--EGKINFDL**W**DLIPFTRGSV | 459 |
| refXP_001216461 | W449 | 439 | DS--NGMINFDL**W**DLIPFTRGST | 459 |
| gbADP03053 | W426 | 416 | DT--AGVASFDV**W**DLLPFTRGYV | 436 |
| gbACR56326 | W448 | 438 | DT--AGVASFDV**W**DLLPFTRGYV | 458 |
| spP13006 | W448 | 438 | DT--AGVASFDV**W**DLLPFTRGYV | 458 |
| gbABG54443 | W424 | 414 | DT--AGVASFDV**W**DLLPFTRGYV | 434 |
| gbAAV68194 | W424 | 414 | DT--AGVASFDV**W**DLLPFTRGYV | 434 |
| gbAAF59929 | W448 | 438 | DT--AGVASFDV**W**DLLPFDRGYV | 458 |
| gbABG66642 | W424 | 414 | DT--AGVASFGV**W**DLLPFTRGYV | 434 |
| embCAC12802 | W447 | 437 | DT--AGAVSFTI**W**DLIPFTRGYV | 457 |
| refXP_001588502 | W448 | 438 | DT--EGAINFDL**W**TLIPFTRGFV | 458 |

Table 10h. Amino acid sequences around W/Y537

| origin | position of mutation | amino acid sequences around mutated position(s) | | |
|---|---|---|---|---|
| gbAAD01493 | W537 | 527 | DYVLQN-FRPN**W**HAVSSCSMMS | 547 |
| gbABM63225 | W537 | 527 | DYVLQN-FRPN**W**HAVSSCSMMS | 547 |
| spQ92452 | W537 | 527 | DYVLQN-FRPN**W**HAVSSCSMMS | 547 |
| refXP_002482295 | W568 | 558 | AYVLQN-FRPN**W**HAVSSCSMMS | 578 |
| embCAE47418 | W537 | 527 | DYVIQN-FRPN**W**HAVSSCSMMA | 547 |
| refXP_002563451 | W536 | 526 | DYVMMN-FRPN**W**HAVSTCSMMS | 546 |
| refXP_001217613 | W561 | 551 | EYVKDN-FRAN**W**HAVGTCSMMS | 571 |
| refXP_001215424 | W535 | 525 | EYVKDN-FRAN**W**HAVGTCSMMS | 545 |
| refXP_001727544 | W538 | 528 | DYVKEN-FRAN**W**HAVSSCSMMS | 548 |
| refXP_002375824 | W538 | 528 | DYVKEN-FRAN**W**HAVSSCSMMS | 548 |
| refXP_001216461 | W539 | 529 | EYVKQN-FRAN**W**HAVSTCAMMS | 549 |
| gbADP03053 | Y515 | 505 | EYIPYH-FRPN**Y**HGVGTCSMMP | 525 |
| gbACR56326 | Y537 | 527 | EYIPYN-FRPN**Y**HGVGTCSMMP | 547 |
| spP13006 | Y537 | 527 | EYIPYH-FRPN**Y**HGVGTCSMMP | 547 |

(continued)

| origin | position of mutation | amino acid sequences around mutated position(s) | | |
|---|---|---|---|---|
| gbABG54443 | Y513 | 503 | EYIPYN-FRPN**Y**HGVGTCSMMP | 523 |
| gbAAV68194 | Y513 | 503 | EYIPYN-FRPN**Y**HGVGTCSMMP | 523 |
| gbAAF59929 | Y537 | 527 | EYIPYH-FRPN**Y**HDVGTCSMMP | 547 |
| gbABG66642 | Y513 | 503 | EYIPYN-FRPN**Y**HGVGTCSMMP | 523 |
| embCAC12802 | Y536 | 526 | EYIKYN-FRPN**Y**HGVGTCSMMK | 546 |
| refXP_001588502 | Y538 | 528 | SYVKQN-FRPN**Y**HNVGSCSMMA | 548 |

Industrial Applicability

The mutated glucose oxidase of the invention may be used for the measurement of glucose which is clinically useful in diagnosis and control of diabetic conditions.

## Claims

**1.** A mutant glucose oxidase modified at one or more positions selected from:

(a) a position corresponding to the position 53 of the amino acid sequence set forth in SEQ ID NO: 1 by substituting the amino acid residue Thr with Ser;
(b) a position corresponding to the position 116 of the amino acid sequence set forth in SEQ ID NO: 1 by substituting the amino acid residue Ile with Val;
(c) a position corresponding to the position 132 of the amino acid sequence set forth in SEQ ID NO: 1 by substituting the amino acid residue Ser with Ala, Thr, Val, Cys or Ile, or by substituting the amino acid residue Thr with Ala, Ser, Val, Trp or Cys;
(d) a position corresponding to the position 134 of the amino acid sequence set forth in SEQ ID NO: 1 by substituting the amino acid residue Thr with Ala, Ile or Met;
(e) a position corresponding to the position 237 of the amino acid sequence set forth in SEQ ID NO: 1 by substituting the amino acid residue Ile with Val, or by substituting the amino acid residue Phe with Ile, Ala, Val, Met, Ser, Asp, Leu, Thr, Asn, Arg or Cys;
(f) a position corresponding to the position 371 of the amino acid sequence set forth in SEQ ID NO: 1 by substituting the amino acid residue Val with Thr, Ala, or by substituting the amino acid residue Ala with Val;
(g) a position corresponding to the position 373 of the amino acid sequence set forth in SEQ ID NO: 1 by substituting the amino acid residue Phe with Leu, Tyr, Ala, Met, Asn or Trp;
(h) a position corresponding to the position 434 of the amino acid sequence set forth in SEQ ID NO: 1 by substituting the amino acid residue Glu with Gln;
(i) a position corresponding to the position 436 of the amino acid sequence set forth in SEQ ID NO: 1 by substituting the amino acid residue Phe with Trp, Ala, Leu, Tyr, Met, Glu or Ile;
(j) a position corresponding to the position 448 of the amino acid sequence set forth in SEQ ID NO: 1 by substituting the amino acid residue Trp with Ala, Ile, Ser, Val, Met, Thr, Cys, Gly, Leu, Asn, Asp, Lys, Phe, Gln or Tyr; and
(k) a position corresponding to the position 537 of the amino acid sequence set forth in SEQ ID NO: 1 by substituting the amino acid residue Trp with Ala.

**2.** The mutant glucose oxidase of claim 1, which has a reduced oxidase activity as compared to the wild-type glucose oxidase.

**3.** The mutant glucose oxidase of claim 1, which has an oxidase activity of less of its dehydrogenase activity.

**4.** The mutant glucose oxidase of claim 1, which has a dehydrogenase activity of 50% or more of the wild-type glucose oxidase.

**5.** The mutant glucose oxidase of claim 1, which has higher ratio of glucose dehydrogenase activity:glucose oxidase activity than that of the wild-type glucose oxidase.

**6.** The mutant glucose oxidase of claim 1 which has the amino acid sequence selected from the group consisting of SEQ ID NOs: 1-21 modified at one or more positions corresponding to the position 53, 116, 132, 134, 237, 371, 373, 434, 436, 448 or 537 of the amino acid sequence set forth in SEQ ID NO: 1.

**7.** An isolated polynucleotide encoding the mutant glucose oxidase of any one of claims 1-6.

**8.** A vector comprising the polynucleotide of claim 7.

**9.** A host cell transformed with a vector of claim 8.

**10.** A method for assaying glucose in a sample, comprising contacting the sample with the glucose oxidase of any one of claims 1-6, and measuring the amount of the glucose oxidized by the glucose oxidase.

**11.** A device for assaying glucose in a sample comprising the glucose oxidase of any one of claims 1-6 and an electron mediator.

**12.** A kit for assaying glucose in a sample comprising the glucose oxidase of any one of claims 1-6 and an electron mediator.

**13.** An enzyme electrode having the glucose oxidase of any one of claims 1-6 which is immobilized on the electrode.

**14.** An enzyme sensor for assaying glucose comprising the enzyme electrode of claim 13 as a working electrode.

**Patentansprüche**

**1.** Mutierte Glukoseoxidase, modifiziert an einer oder mehreren Positionen ausgewählt aus:

(a) einer Position, die der Position 53 der Aminosäuresequenz gemäß SEQ ID NO: 1 entspricht, durch Substituieren des Aminosäurerests Thr mit Ser;
(b) einer Position, die der Position 116 der Aminosäuresequenz gemäß SEQ ID NO: 1 entspricht, durch Substituieren des Aminosäurerests Ile mit Val;
(c) einer Position, die der Position 132 der Aminosäuresequenz gemäß SEQ ID NO: 1 entspricht, durch Substituieren des Aminosäurerests Ser mit Ala, Thr, Val, Cys oder Ile oder durch Substituieren des Aminosäurerests Thr mit Ala, Ser, Val, Trp oder Cys;
(d) einer Position, die der Position 134 der Aminosäuresequenz gemäß SEQ ID NO: 1 entspricht, durch Substituieren des Aminosäurerests Thr mit Ala, Ile oder Met;
(e) einer Position, die der Position 237 der Aminosäuresequenz gemäß SEQ ID NO: 1 entspricht, durch Substituieren des Aminosäurerests Ile mit Val oder durch Substituieren des Aminosäurerests Phe mit Ile, Ala, Val, Met, Ser, Asp, Leu, Thr, Asn, Arg oder Cys;
(f) einer Position, die der Position 371 der Aminosäuresequenz gemäß SEQ ID NO: 1 entspricht, durch Substituieren des Aminosäurerests Val mit Thr, Ala oder durch Substituieren des Aminosäurerests Ala mit Val;
(g) einer Position, die der Position 373 der Aminosäuresequenz gemäß SEQ ID NO: 1 entspricht, durch Substituieren des Aminosäurerests Phe mit Leu, Tyr, Ala, Met, Asn oder Trp;
(h) einer Position, die der Position 434 der Aminosäuresequenz gemäß SEQ ID NO: 1 entspricht, durch Substituieren des Aminosäurerests Glu mit Gln;
(i) einer Position, die der Position 436 der Aminosäuresequenz gemäß SEQ ID NO: 1 entspricht, durch Substituieren des Aminosäurerests Phe mit Trp, Ala, Leu, Tyr, Met, Glu oder Ile;
(j) einer Position, die der Position 448 der Aminosäuresequenz gemäß SEQ ID NO: 1 entspricht, durch Substituieren des Aminosäurerests Trp mit Ala, Ile, Ser, Val, Met, Thr, Cys, Gly, Leu, Asn, Asp, Lys, Phe, Gln oder Tyr; und
(k) einer Position, die der Position 537 der Aminosäuresequenz gemäß SEQ ID NO: 1 entspricht, durch Substituieren des Aminosäurerests Trp mit Ala.

**2.** Mutierte Glukoseoxidase nach Anspruch 1, die im Vergleich zur Wildtyp-Glukoseoxidase eine reduzierte Oxidaseaktivität aufweist.

3. Mutierte Glukoseoxidase nach Anspruch 1, die eine Oxidaseaktivität von weniger als ihrer Dehydrogenaseaktivität aufweist.

4. Mutierte Glukoseoxidase nach Anspruch 1, die eine Dehydrogenaseaktivität von 50% oder mehr der Wildtyp-Glukoseoxidase aufweist.

5. Mutierte Glukoseoxidase nach Anspruch 1, die ein höheres Verhältnis von Glukosedehydrogenaseaktivität zu Glukoseoxidaseaktivität als das der Wildtyp-Glukoseoxidase aufweist.

6. Mutierte Glukoseoxidase nach Anspruch 1, die die aus der aus SEQ ID NO: 1-21 bestehenden Gruppe ausgewählte Aminosäuresequenz aufweist, die an einer oder mehreren Positionen modifiziert ist, die der Position 53, 116, 132, 134, 237, 371, 373, 434, 436, 448 oder 537 der Aminosäuresequenz gemäß SEQ ID NO: 1 entsprechen.

7. Isoliertes Polynukleotid, codierend die mutierte Glukoseoxidase nach einem der Ansprüche 1-6.

8. Vektor, umfassend das Polynukleotid nach Anspruch 7.

9. Wirtszelle, transformiert mit einem Vektor nach Anspruch 8.

10. Verfahren zum Bestimmen von Glukose in einer Probe, das Inkontaktbringen der Probe mit der Glukoseoxidase nach einem der Ansprüche 1-6 und Messen der Menge der von der Glukoseoxidase oxidierten Glukose umfasst.

11. Vorrichtung zum Bestimmen von Glukose in einer Probe, umfassend die Glukoseoxidase nach einem der Ansprüche 1-6 und einen Elektronenvermittler.

12. Kit zum Bestimmen von Glukose in einer Probe, umfassend die Glukoseoxidase nach einem der Ansprüche 1-6 und einen Elektronenvermittler.

13. Enzymelektrode, aufweisend die Glukoseoxidase nach einem der Ansprüche 1-6, die auf der Elektrode immobilisiert ist.

14. Enzymsensor zum Bestimmen von Glukose, umfassend die Enzymelektrode nach Anspruch 13 als Arbeitselektrode.

## Revendications

1. Glucose oxydase mutante modifiée à une ou plusieurs positions sélectionnées parmi :

(a) une position correspondant à la position 53 de la séquence d'acides aminés exposée dans SEQ ID n° : 1 en substituant le résidu d'acide aminé Thr par Ser ;
(b) une position correspondant à la position 116 de la séquence d'acides aminés exposée dans SEQ ID n° : 1 en substituant le résidu d'acide aminé Ile par Val ;
(c) une position correspondant à la position 132 de la séquence d'acides aminés exposée dans SEQ ID n° : 1 en substituant le résidu d'acide aminé Ser par Ala, Thr, Val, Cys ou Ile, ou en substituant le résidu d'acide aminé Thr par Ala, Ser, Val, Trp ou Cys ;
(d) une position correspondant à la position 134 de la séquence d'acides aminés exposée dans SEQ ID n° : 1 en substituant le résidu d'acide aminé Thr par Ala, Ile ou Met ;
(e) une position correspondant à la position 237 de la séquence d'acides aminés exposée dans SEQ ID n° : 1 en substituant le résidu d'acide aminé Ile par Val, ou en substituant le résidu d'acide aminé Phe par Ile, Ala, Val, Met, Ser, Asp, Leu, Thr, Asn, Arg ou Cys ;
(f) une position correspondant à la position 371 de la séquence d'acides aminés exposée dans SEQ ID n° : 1 en substituant le résidu d'acide aminé Val par Thr, Ala, ou en substituant le résidu d'acide aminé Ala par Val ;
(g) une position correspondant à la position 373 de la séquence d'acides aminés exposée dans SEQ ID n° : 1 en substituant le résidu d'acide aminé Phe par Leu, Tyr, Ala, Met, Asn ou Trp ;
(h) une position correspondant à la position 434 de la séquence d'acides aminés exposée dans SEQ ID n° : 1 en substituant le résidu d'acide aminé Glu par Gln ;
(i) une position correspondant à la position 436 de la séquence d'acides aminés exposée dans SEQ ID n° : 1 en substituant le résidu d'acide aminé Phe par Trp, Ala, Leu, Tyr, Met, Glu ou Ile ;

(j) une position correspondant à la position 448 de la séquence d'acides aminés exposée dans SEQ ID n° : 1 en substituant le résidu d'acide aminé Trp par Ala, Ile, Ser, Val, Met, Thr, Cys, Gly, Leu, Asn, Asp, Lys, Phe, Gin ou Tyr ; et
(k) une position correspondant à la position 537 de la séquence d'acides aminés exposée dans SEQ ID n° : 1 en substituant le résidu d'acide aminé Trp par Ala.

**2.** Glucose oxydase mutante selon la revendication 1, qui a une activité oxydase réduite telle que comparée à la glucose oxydase de type sauvage.

**3.** Glucose oxydase mutante selon la revendication 1, qui a une activité oxydase inférieure à son activité déshydrogénase.

**4.** Glucose oxydase mutante selon la revendication 1, qui a une activité déshydrogénase de 50 % ou plus de la glucose oxydase de type sauvage.

**5.** Glucose oxydase mutante selon la revendication 1, qui a un rapport supérieur de l'activité glucose déshydrogénase:activité glucose oxydase par rapport à celui de la glucose oxydase de type sauvage.

**6.** Glucose oxydase mutante selon la revendication 1 qui a la séquence d'acides aminés sélectionnée parmi le groupe constitué de SEQ ID $n^{os}$ : 1 à 21 modifiée à une ou plusieurs positions correspondant à la position 53, 116, 132, 134, 237, 371, 373, 434, 436, 448 ou 537 de la séquence d'acides aminés exposée dans SEQ ID n° : 1.

**7.** Polynucléotide isolé codant pour la glucose oxydase mutante selon l'une quelconque des revendications 1 à 6.

**8.** Vecteur comprenant le polynucléotide selon la revendication 7.

**9.** Cellule hôte transformée avec un vecteur selon la revendication 8.

**10.** Procédé de test du glucose dans un échantillon, comprenant la mise en contact de l'échantillon avec la glucose oxydase selon l'une quelconque des revendications 1 à 6, et la mesure de la quantité du glucose oxydé par la glucose oxydase.

**11.** Dispositif de test du glucose dans un échantillon comprenant la glucose oxydase selon l'une quelconque des revendications 1 à 6 et un médiateur d'électrons.

**12.** Trousse de test du glucose dans un échantillon comprenant la glucose oxydase selon l'une quelconque des revendications 1 à 6 et un médiateur d'électrons.

**13.** Électrode à enzyme ayant la glucose oxydase selon l'une quelconque des revendications 1 à 6 qui est immobilisée sur l'électrode.

**14.** Capteur d'enzyme pour le test du glucose comprenant l'électrode à enzyme selon la revendication 13 comme électrode de travail.

Fig. 1

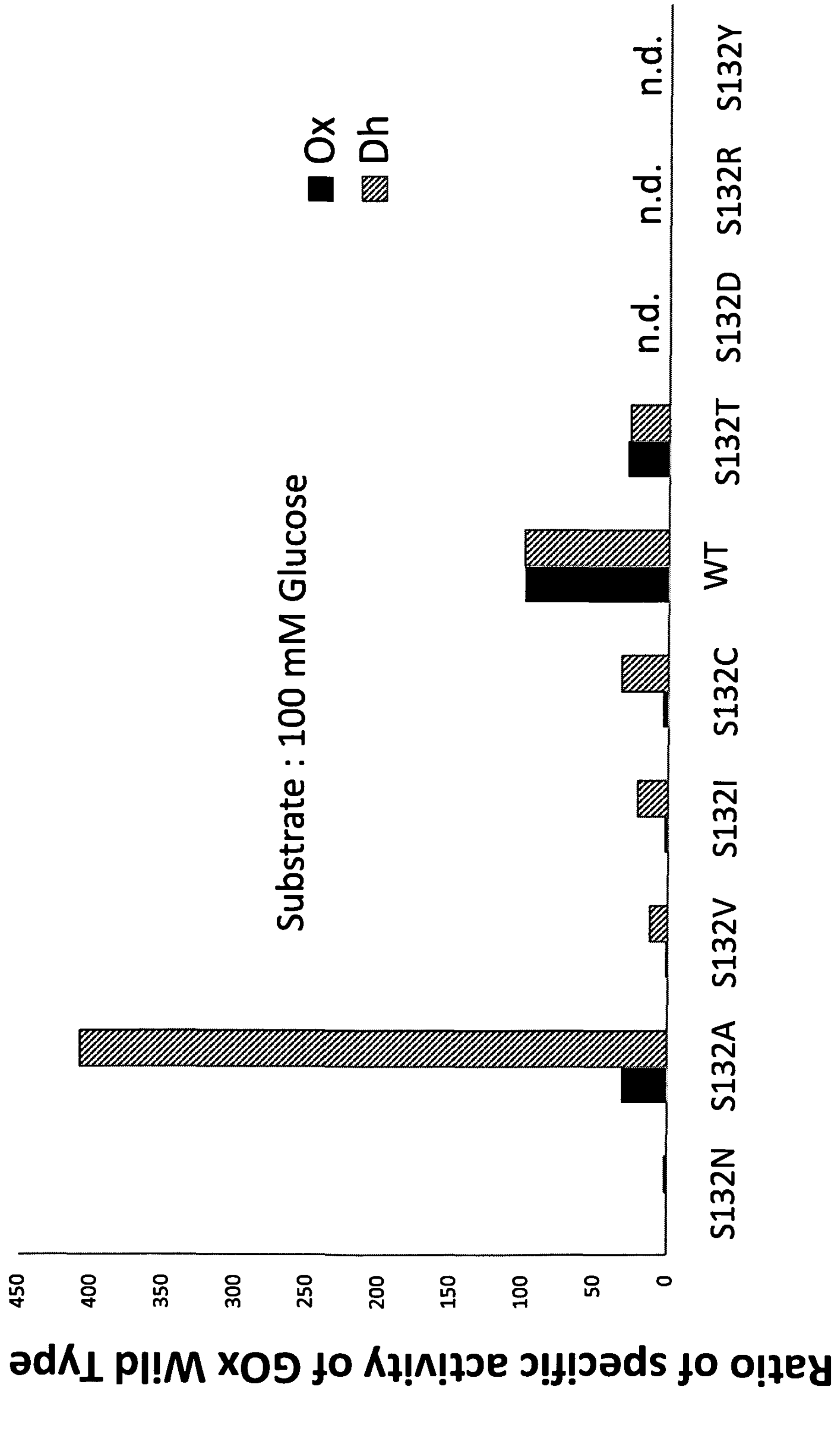
Substrate : 100 mM Glucose
Ox
Dh
Ratio of specific activity of GOx Wild Type (%)
0
50
100
150
200
250
300
350
400
450
S132N
S132A
S132V
S132I
S132C
WT
S132T
S132D
S132R
S132Y
n.d.
n.d.
n.d.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2004113900 A
- US 5997817 A
- US 7008799 B
- US 6036919 A
- US 5334508 A

### Non-patent literature cited in the description

- **LU, G. ; MORIYAMA, E. N.** Vector NTI, a balanced all-in-one sequence analysis suite. *Brief Bioinform,* 2004, vol. 5, 378-88